# NEW EUROPEAN PATENT SPECIFICATION

(11) **EP 0 374 730 B2**
(45) Date of publication and mention of the opposition decision: **19.04.2000**
(45) Mention of the grant of the patent: 06.04.1994
(21) Application number: 89123131.8
(22) Date of filing: 14.12.1989
(51) Int. Cl.: A61F 13/56

(54) **Fastening system for disposable diaper with disposability feature**
Verschluss mit einem wegwerfbaren Element für eine Wegwerfwindel
Système de fermeture pour couche de bébé à jeter pourvu d'un élément jetable

(30) Priority: 20.12.1988 US 287746
(43) Date of publication of application: 27.06.1990
(60) Divisional application: 93103678.4
(73) Proprietor: KIMBERLY-CLARK WORLDWIDE, INC., Neenah, Wisconsin 54956 (US)
(72) Inventor: Roessler, Thomas Harold, Wisconsin 54925 (US); Siebers, Bruce Michael 60 Regal Terrace, Wisconsin 54915 (US); Popp, Robert Lee, Wisconsin 54944 (US); Fallen, Charles Rayburn 1658 Tonya Trail, Wisconsin 54956 (US)
(74) Representative: Grünecker, Kinkeldey, Stockmair & Schwanhäusser Anwaltssozietät

(56) References cited:
- EP-A- 0 048 446
- EP-A- 0 187 725
- EP-A- 0 235 014
- EP-A- 0 240 213
- EP-A- 0 321 232
- EP-A- 0 321 234
- WO-A-83/03754
- GB-A- 2 129 689
- US-A- 3 089 494
- US-A- 3 150 664
- US-A- 3 869 761
- US-A- 3 955 575
- US-A- 4 047 529
- US-A- 4 681 581

## Description

The present invention relates to an absorbent article of the type as defined in the preamble of claim 1.

An absorbent article of this type is disclosed in WO-A-83/037 54. The known absorbent article is a diaper comprising a rectangular washable diaper form, the smaller sides of which form the waist portions. The diaper form comprises a primary fastening means consisting of two first components and a plurality of second components. The first components are arranged as laterally extending tabs adjacent with one of the waist portions. The plurality of second components are arranged adjacent or slightly inwards of the opposite waist portion, in the second half of the diaper form when folded along its transverse centre line, opposite the first half which contains the first fastening tabs. The plurality of second components shall allow the diaper to be adapted to different sizes. The diaper does not have a secondary fastening means adapted to secure the diaper and the waste therein in a disposal condition.

US-A-3150664 describes an adjustable diaper having a primary fastening means for attaching the diaper to the body of the wearer. This fastening means includes two first fastening components attached to one half of the diaper and a plurality of second components attached to the second half-portion of the diaper. Each of the second components being alternatively engaged by the first components to fit the diaper to the specific size of an infant. Secondary fastening means for securing the diaper and the waste therein in a disposal condition are not mentioned.

US-A-3869761 describes a diaper which can be rolled up in to a condition for disposal. The diaper has a primary fastening means consisting of a pair of adhesively coated tabs arranged at one waistband portion of the diaper and adapted to be stuck to the diaper body if the diaper is attached to the body of the user. The diaper further has a secondary fastening means consisting of two further adhesively coated tabs which are covered in use of the diaper by the tabs of the primary fastening system. When the diaper is to be disposed, the tabs of the primary fastening system are to be torn off the diaper, thus exposing the adhesive coating of the tabs of the secondary fastening system to be attached to the body of the diaper if the diaper is in rolled up condition for disposal. The publication does not describe a secondary fastening system to be used with a mechanical primary system.

Further fastening systems heretofore in use have included adhesive fastening, such as shown, in US-A-3,180,355; US-A-3,630,201; US-A-4,047,530, US-A 4,049,001; US-A-4,050,453 and FR-A-7,436,169. The fastening device generally comprises an adhesive tab attached to the outer (backing) sheet of the diaper at the rear portion and the active adhesive surface of the tab is stuck on to another portion of the backing sheet surface near to the front portion thereby closing the diaper. The adhesive system for the primary fastening system of the waistband around the wearer is susceptible to contamination of either the adhesive tab surface or the cover surface to which the tab is applied. Recently, mechanical closure systems have been devised using hook (e.g."mushroom") and loop (for example Velcro^{R}) fasteners for closure of the waistband as set forth by FR-A- 2, 594,650 and EP-A 0 276 970.

It will therefore, be appreciated that there has been a significant need for a fastening system for a disposable article which easy in design and which is able to securely seal a soiled absorbent article after use in a rolled or folded condition for disposal.

An absorbent article fulfilling this need is described in claim 1.

In accordance with the present invention, a first component of a primary fastening means used to hold the absorbent article on a user, are combined with a common third component constructed to mechanically engage each of the first components.

In a preferred embodiment the primary and secondary fastener systems utilize hook and loop fastener components.

Further developments of the invention are described in the dependent claims 3 to 19.

Embodiments of the invention are hereinafter described by reference to the figures, wherein:
FIG. 1 is a top plan view of a disposable diaper constructed according to an embodiment of the invention;
FIG. 2 is a sectional view (somewhat schematic) taken along line 2-2 on FIG. 1;
FIG. 3 is a sectional elevational view (somewhat schematic) taken along line 3-3 on FIG. 1;
FIG. 4 is a perspective view of the diaper of FIG. 1 folded and fastened for disposal;

The absorbent article illustrated by the embodiment shown on the drawings utilizes a first fastener means of the mechanical type positioned at one of the longitudinal ends of the garment which comprise one waistband portion of the article. The other cooperating waistband portion is at the opposite longitudinal end of the garment; the two waistband portions encircling the waist of the user in overlapping fashion. The mechanical type fastener illustrated includes two cooperating components mounted on the waistband portions that interengage one another in the overlapping of the waistband portions to releasably fasten the waistband in an adjusted, snug fit on the wearer. One component is a hook style swatch or tab and the second component is a loop style patch.

In the present invention, a second fastener means is included and positioned on the article to enable rolling or folding the absorbent article after use into a neat bundle for subsequent handling and disposal. This second fastener means may utilize the one component placement on the waistband of the first fastener and combines with it a separate and compatible third component in the same longitudinal half of the article such that two components of the second fastener means are engaged to hold the rolled or folded article in its disposability condition.

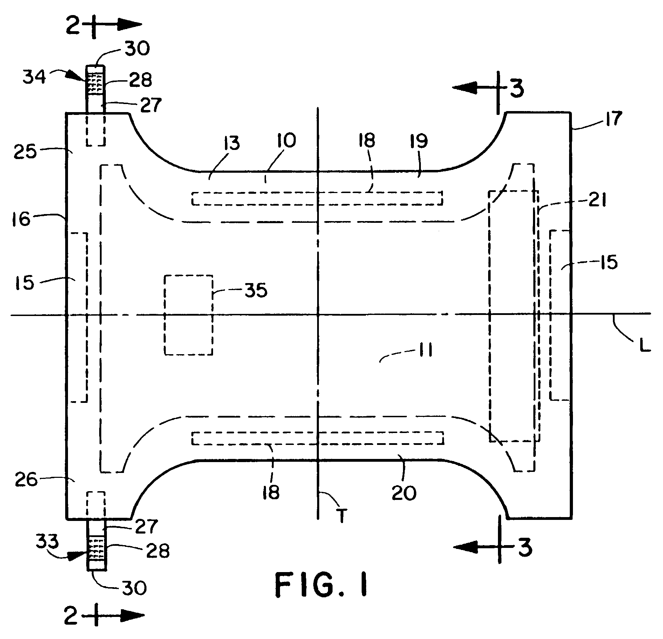

Referring to FIGS. 1-4, an absorbent article is constructed which has a primary mechanical fastener for the waistband of the article and a secondary fastener providing the disposability feature. The article includes an elongated backsheet 10 constructed of a layer of 25,4 µm (1 mil) polyethylene and TiO₂ filler which is overlaid with absorbent body 11, such as an absorbent body comprising 78% airlaid cellulose fiber and 11% hydrogel, enclosed in a 9% tissue wrap 12. The absorbent body has a density on the order of 0.12 grams per cubic centimeter. The absorbent body 11 is overlaid with a liquid permeable topsheet 13 corresponding in shape to backsheet 10. An example of the topsheet is a 25,4 g/m² (0.75 ounce per square yard) polypropylene spunbond material. The backsheet 10 and topsheet 13 are fastened together peripherally by a spray application of adhesive 14, such as National 70-3016 adhesive. The tissue wrap 12 is likewise adhesively attached to the inside of backsheet 10.

A suitable hook material may, for example, be produced from a process of continuous injection molding of a polymeric material, such as a polypropylene copolymer. It has been determined that the proper stiffness of the material is obtained from the copolymer having flexural modulus of 483 to 828 N/mm² (70,000 - 120,000 psi) and shore hardness value within the range of about D-40 to D-80, preferably about D-61.

The base strata of the hook material is fabricated onto the surface of the tab 30 by adhesive, heat-bonding or sonic-welding to provide the J-shaped hooks in a configuration facing outwardly of the tab structure. The hooks are tapered base to top with an alternating hook design. A specific example of a suitable hook is the HTH #707 available from Velcro U.S.A. The overall thickness (caliper) of the hook material is in the range of 0,085 - 0,13 cm (.035 to .050 inch), and preferably about 0,11 cm (0.045 inch). The hooks are attached to a base film which is in the range of 0,013 - 0,064 cm (.005 to .025 inch) thick, preferably a thickness in the range of about 0,0203 - 0,0254 cm (.008 to .010 inch). The hook density on the base is within the range of 68 - 161 hooks per cm² (440 to 1040 hooks per square inch); preferably a hook density of about 114 hooks per cm² (740 hooks per square inch), as a particular example, hook material having a row density within the range or about 7,9 - 23,6 rows to the lineal cm (20-60 rows to the lineal inch) of width, preferably a row density of about 16 rows per cm (40 rows per lineal inch). The material used may be clear or opaque with selected color of the material.

A representative loop component of the hook and loop fastener is a fabric material of raised loop construction in which the fabric is stabilized (loops are erect from the fabric's base) through napping. Additionally, the loop material may be thermoset to impart other properties, such as is set forth in US-A-3,475,926 and US-A-3,090,097.

Preferably, a material of polyolefin-based fibers or other synthetic fibers is used; an example of the fabric being a tricot polyester. More specifically, polyethylene terephthalate (PET) fiber mix in which about 15 - 35% of the fabric yarns are yarns composed of 1-15 filaments with the yarn having a linear density within the range of about 1,67 - 3,3 tex (15-30d), and about 65 - 85% of the fabric yarns are yarns having about 10-30 filaments per yarn and having a yarn linear density within the range of about 3,3 - 5,6 tex (30-50d) comprises the fibers used in the fabric. Two bar warp knit construction is preferred in which courses are in the range 8,27 - 16,14 per cm (21 - 41 per inch) and wales are in the range 10,27 - 18,11 per cm (26 - 46 per inch). The surface of the fabric is napped. The thickness caliper is within the range of about 0,254 to 1,02 mm (.01 to .04 inch), the preferred caliper being about 0,89 mm (.035 inch). The basis weight of the fabric is in the range of about 34 to 102 g/m² (1.0 to 3.0 ounces per square yard); the preferred basis weight being about 54,4 g/m² (1.6 ounces per square yard). The fabric is printable or decoratable directly, or in the alternative may be laminated over a predecorated film base, whereby a print pattern shows through the loop material. Loop material is applied onto the front waistband portion 17 in a patch or swatch 21 with the loops erect and facing outwardly from the face of the backsheet 10. The extent of patch 21 may be varied and may take one of several geometric shapes, such as rectangular, irregular shape, diamond, triangle, circle, oval, chevron or the like. The loop material may also be configured as a plurality of patches. The plural patches (two or more) form a landing zone for the hook tabs which are composed of multiple, separate sections. The variation of shape or configuration of multiple patches will lend itself to desired function and size for fastening the waistband about the various sizes of users.

Waist elastic members 15 (shown schematically on FIG. 1) are attached along the opposite waistband portions 16 and 17 at the opposite longitudinal ends of the article. Preferably, the members 15 are at the outside margins of the waistbands 16 and 17, respectively. The elastic members are arranged to gather and shirr the waistbands 16 and 17 of the garment to provide a seal about the waist of the wearer. Also, leg elastic members 18 are attached to the opposite side margins 19 and 20 of the article and arranged to gather and shirr these side margins to provide seals about the legs of the wearer.

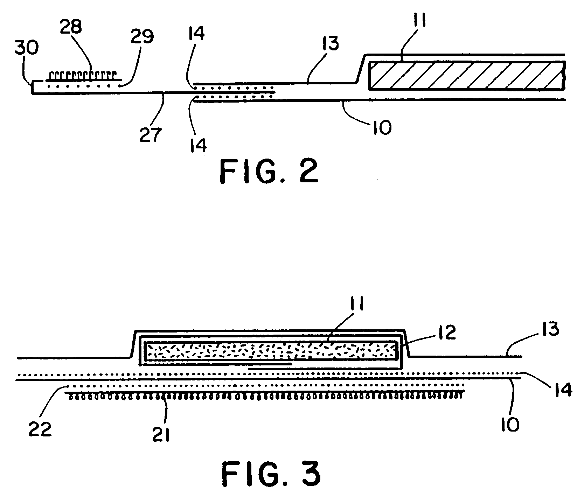

A first fastener means is provided on the garment comprising a fastening patch of loop material 21. The loop material 21 is one of the two components of the primary fastener means, and it interengages a second component in the form of hook materials, to be presently described. Loop material 21 may comprise a woven or nonwoven material. Preferably, the loop material is a Guilford warp knit fabric, number 19902, 54 g/m² (1.57 ounces per square yard), made from polyester fiber. Loop patch 21 is, for example, attached by adhesive 22 (FIG. 3) to the outside surface of backsheet 10 just below and interiorly of the elastic member at waistband 17. Patch 21 may alternatively extend to the terminal edge of the waistband. The loop material patch 21 may be adhesively fastened using National 70-3016 hot melt adhesive available from National Starch Company, in Bridgewater, New Jersey. In normal use, waistband 17 is disposed at the front of the garment article and waistband 16 is the rear portion which includes the ear portions 25 and 26. Waistband 16 wraps around the back of the torso and overlaps onto the front waistband 17; however, the garment may be constructed for encircling the waist in the reverse order. For the sake of description and illustration only, the front and rear waistbands 17 and 16, respectively, are identified in accordance with the conventional arrangement and use of the garment.

At the end portion of the article opposite the patch 21, [in this case, at the rear waistband 16] two fastener tabs 33 and 34 are attached securely to the backsheet to extend outwardly from the opposite ear portions 25 and 26, (Fig. 1), respectively, of the garment. Tabs 33 and 34 are each constructed of a tape 27 composed, for example, of a plastic film substrate, such as a 0,11 mm (4.5 mil) polypropylene having TiO₂ filler. As illustrated in Fig. 2, the tabs each have end segments of the film sandwiched between the backsheet 10 and topsheet 13 in the opposite ear portions 25 and 26 along the sides of the article and the film substrate is attached thereat by a hot melt adhesive, such as National 70-3016 hot melt adhesive. The secured overlap of the tape 27 with backsheet 10 and topsheet 13 is approximately 25,4 mm (1.0 inch) measured inwardly from the margin of the ear portion; however, the overlap may be from 6,35 to 101,6 mm (0.25 to 4.0 inches). A fastener component, such as a region of hook material 28, is incorporated onto the film substrate to face upwardly in the direction of top sheet 13. In the illustrated embodiment, the hook material 28 is attached to the film substrate with hot melt adhesive 29 (National 70-3016 being a satisfactory adhesive for this purpose). In an alternative embodiment, the hook material may be integrally formed with the film substrate. Hook material 28 may comprise Velcro^{R} hook "style 15" and may be composed of a polypropylene, such as Telcar 102 polypropylene distributed by Teknor Apex Co. of Pawtucket, Rhode Island, or Ferro polypropylene 9004N distributed by Horizon Polymers of Houston, Texas. A finger tab portion 30 is exposed at the free outboard end of the attaching tape 27 for ease in grasping the tab and releasing the hook component once it is affixed to the loop component.

The garment is placed on the body of the wearer and front waistband 17 is overlapped by rear waistband 16 bringing the ear portions toward each other to a firm or snug fit. The hook material 28 on tabs 33 and 34 are pressed onto and engage the loop material of patch 21 facing outwardly on the backsheet at the front waistband mid-portion. The primary hook and loop fastener of the type described for closing the waistband and holding it in service about the wearer should have properties of shear force in the range of 0,046 to 0,138 N/mm² (6.6 - 20 psi), and peel force in the range of 7,9 to 47,2 g/mm (200 to 1200 grams per lineal inch) of transverse width. A preferred peel force value is within the range or about 15,75 to 23,62 g/mm (400-600 grams per inch) width.

"Shear" is determined according to ASTM Designation: D3654-82, "Standard Test Method for Holding Power of Pressure-Sensitive Tapes", which is incorporated herein by reference, and subject to the following modifications: In relation to the test, the closure is placed under an increasing load. The system being tested is a hook and loop closure system. (See 1. Scope). The apparatus should include an "INSTRON" or equivalent continuous rate of extension (CRE) tensile tester. (See 3. Apparatus). In carrying out the procedure (See 6. Procedure), test direction of the materials should be noted. The test materials are rolled five cycles 6,45 cm² or (1 sq. in.), where one cycle equals once in each direction. The hook material is clamped into the lower jaw of the Instron tensile tester. The engaged system (hook and loop) is pulled until failure. In doing the calculations (See 10. Calculations), the peak load is determined and recorded in grams.

"Peel" is determined according to ASTM Designation: D1876-72, "standard Test Methods for Peel Resistance of Adhesives (T-Peel Test)", which is incorporated herein by reference, and subject to the following modifications:

4.1 No test panels are used; hook and loop materials are directly engaged and are not mounted on any other substrate unless specified. Test direction of the material should be noted. No panels are used. The engaged test materials are rolled five cycles; where one cycle equals once in each direction. The hook material is clamped into the upper jaw and the loop material is clamped into the lower jaw.

In a suitable hook and loop fastening system, the fastener has a total peel resistance of at least about 150 gm. and preferably has a total peel resistance of at least about 400 gm. The total shear force resistance is at least about 750 gm. and preferably is at least about 1000 gm. It should be readily recognized that a suitable fastening system will include a selected balance between the property of total peel resistance and the property of total shear force resistance. For example, a system with the lower values of peel resistance could be more suitable if the system also exhibited a higher total shear force resistance.

For the purposes of the present description, the total peel resistance value corresponds to the peel force determined in accordance with ASTM D1876-72 multiplied by the transverse width of engagement between the hook material and the loop material employed in the particular fastening system. Similarly, the total shear force resistance value corresponds to the shear stress determined in accordance with ASTM D3654-82 multiplied by the area of engagement between the hook material and loop material of the fastening system.

A second loop material patch 35 as a third component is adhesively attached to the backsheet 10 facing outwardly and disposed in the same longitudinal half of the diaper with ears 25 and 26. National 70-3016 hot melt adhesive may be used to apply the patch 35 to the polymer backsheet. The patch 35 is constructed of a loop material, an example of which is Guilford warp knit fabric 19902 made of polyester. Patch 35 is placed such that it is centered on the longitudinal axis L and is generally in the back half of the diaper, that is the half of the diaper left of the transverse center line T on FIG. 1.

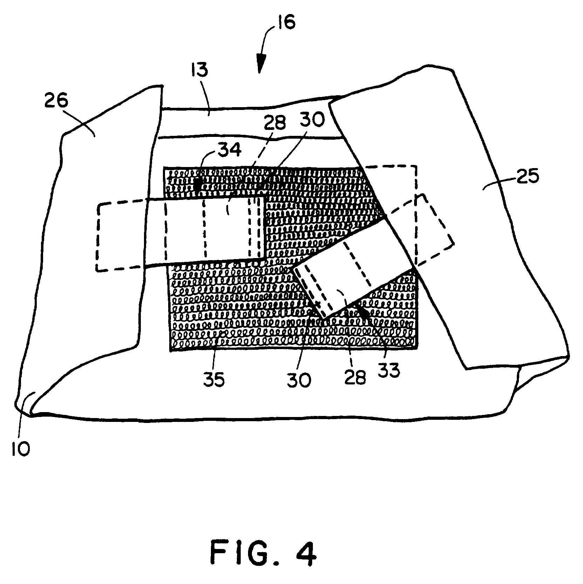

Referring to FIG. 4, the garment may be disposed of after use by folding or rolling it inwardly from the front waistband 17 beyond the transverse center T (FIG. 1) such that the patch 35 is generally disposed between ear portions 25 and 26 and facing outwardly. The tabs 33 and 34, which were part of the primary fastening system, are brought inwardly and toward each other so that the hook material 28 of each overlies the loop material of patch 35 and then pressed into engagement. The hooks 28 of tabs 33 and 34 attach to loops of patch 35 and form a secure secondary fastener holding the used garment in its rolled or folded condition for convenient disposal.

As was earlier mentioned, the hook and loop fastener of this secondary fastening system should have properties in shear of about 0,3 g/mm² (200 grams per square inch) (minimum), and peel about 2,9 g/mm (75 grams per lineal inch) width (minimum). These properties of shear and peel may be less than that employed in the primary fastening means such as the tabs 33 and 34 are seated on patch 21 for securing the garment around the waist of a wearer. The forces applied against the primary fastening of the waistband are greater while in service on the wearer; whereas, once rolled for disposal the garment needs meet only the forces tending to open up or unroll from the disposal form.

A preferred example of the absorbent article of this second embodiment is a disposable diaper, such as shown on FIGS. 1 and 4. The dimensions for the components of the primary and secondary fastening means are the following:

| Part Name | No. | Range Width [x 2,54 cm] | Range Length [x 2,54 cm] | Preferred Dimensions or Medium Diaper | |
|---|---|---|---|---|---|
| | | | | Width [x 2,54 cm] | Length [x 2,54 cm] |
| Loop Patch | 21 | .5 - 5 | 4 - 14 | 1.75 | 9 |
| Fastening Tape | 27 | .25 - 4 | 1 - 6 | 1 | 3 |
| Hook Material | 28 | .25 - 4 | .1 - 4 | 1 | .5 |
| Disposal Loop Patch | 35 | .25 - 5 | .25 - 5 | 2.5 | 3 |

In the disclosure, the elements are disclosed as being attached by adhesives, as the preferred examples for connecting the various fastener elements to the sheets and tapes of the article. It should be understood these attachments may be made by sonic welding, ultrasonic techniques, heat bonding (where applicable) or other known means of attachment.

In selecting a style of mounting the attachment tab 27 onto the article, certain trade-offs are available comparing security or strength of the attachment versus cost in adhesives, material and manufacturing cost of application.

In connection with the embodiments described herein and within the concept of the invention, the hook and loop mechanical fastener can be replaced with other mechanical fastening systems (not shown), such as buttons, hooks, hook and eye fasteners, snaps, or the like; cooperating with corresponding buttonholes, eye receivers, snap receivers or the like. While the representative embodiments of the invention described above include specific locations for the hook components and the loop components of the fastening systems, it is readily apparent that the relative locations of the hook and loop components may be reversed or exchanged for each other. In optional arrangements, a hook component may be substituted for the described loop component, and a loop component may be substituted for the described hook component to produce an alternative, operative configuration.

## Claims

1. An absorbent article comprising:
superimposed backsheet (10), absorbent body (11) and topsheet (13) elements formed to a composite;
opposite longitudinal end portions of said composite providing first and second waistband portions (16,17), respectively, adapted to overlap each other near their lateral ends upon encircling the abdomen of the user;
primary mechanical fastening means (27) for securing said first and second waistband portions (16,17) to each other in said overlapping relationship, said primary mechanical fastening means (27) having a first component (28) located in a first longitudinal half-portion of said composite and attached to said composite at each lateral end of said first waistband portion (16) facing inwardly of said article and having a second component (21) attached to said composite at said second waistband portion (17), said second component (21) constructed to cooperatively interengage said first component (28) and releasably fasten thereto;
**characterized by** a combination therewith of secondary mechanical fastening means for securing the composite in a disposal condition after use, said secondary mechanical fastening means including an outwardly facing third component (35) arranged as a single element and overlying said backsheet (10) in said first half-portion of said composite, said third component (35) being constructed to mechanically engage each of said laterally positioned first components (28) to secure the composite in the disposal condition with each of said first components (28) overlying said third component (35).

2. The absorbent article of claim 1 in which the primary mechanical fastener means comprises:
a hook and loop fastener comprising two interlocking materials, one being a hook material and the other being a loop material;
one of said two materials providing said first component (28) attached to the lateral end portions of said first waistband portion (16) and another of said two material providing said second component (21) attached to said second waistband portion (17), the said material being engageable to attach the first and second waistband portions (16,17) to each other.

3. The absorbent article of claim 1 or 2, wherein the third component (35) is fixedly attached to the backsheet (10) and centered on a longitudinal axis (L) therof.

4. An article as recited in any preceding claim, wherein said first component (28) is a polymeric, hook style material having a flexural modulus of 482,600 - 827,440 kPa (70,000 - 120,000 psi) and a Shore hardness within a range of D-40 to D-80.

5. An article as recited in claim 4, wherein said Shore hardness is about D-61.

6. An article as recited in any preceding claim, wherein the first component (28) includes a hook material having an alternating hook design.

7. An article as recited in any preceding claim, wherein said first component (28) includes a hook material having a hook density within a range of 440 to 1040 hooks per square inch (68 - 161 hooks per square cm).

8. An article as recited in claim 7, wherein said hook material has a hook density of about 740 hooks per square inch (about 114 hooks per square cm).

9. An article as recited in any preceding claim, wherein said component (28) includes a hook material having a row density within a range of 7,9 - 23,6 rows to the lineal cm of width (20 - 60 rows to the lineal inch of width).

10. An article as recited in claim 9, wherein said hook material has a row density of about about 16 rows to the lineal cm of width (40 rows to the lineal inch of width).

11. An article as recited in any preceding claim, wherein said second component (21) includes a loop material comprising a two bar warp knit fabric having courses in a range of 8 - 16 per cm (21 - 41 per inch) and having wales in a range of 10-18 per cm (26-46 per inch).

12. An article as recited in any preceding claim, wherein said second component (21) includes a loop material having a caliper within a range of 0,025 to 0,1 cm (0.01 0.04 inch).

13. An article as recited in any preceeding claim, wherein said second component (21) includes a loop material having a basis weight within a range of 34-102 gm per square meter (1.0-3.0 ounces per square yard).

14. An article as recited in any preceding claim, wherein said first component (28) comprises a hook material (28) having a polymeric base material of 0,0203 - 0,0254 cm (0.008 - 0.01 inch) thickness and a hook configuration which is tapered base to top.

15. An article as recited in any preceding claim wherein said first component (28) comprises a hook material (28) having a caliper in the range of 0,089 - 0,127cm (0.035 to 0.050 inch).

16. An article as recited in any preceding claim, wherein said second component (21) comprises a loop material composed of a knit fabric having a raised and stabilized loop construction.

17. An article as recited in any preceding claim, wherein said second component (21) comprises a fabric loop material composed of synthetic fibers, said fabric including fabric yarns in which about 15% - 35% of the fabric yarns are yarns having 1 - 15 filaments per yarn and having a yarn linear density within the range of about 1,67 - 3,3 tex (15 - 30d), and in which about 65% - 85% of the fabric yarns are yarns having about 10 - 30 filaments per yarn and a yarn linear density within the range of about 3,3-5,6 tex (30 - 50d).

18. An article as recited in any preceding claim wherein said first component (28) and third component (35) have a minimum shear property therebetween of about 31 gm per square cm (about 200 gm per square inch) and a minimum peel property of about 30gm per cm of width (about 75 grams per lineal inch of width).

19. An article as recited in claim 17, wherein said synthetic fibers are composed of polyethylene terephthalate (PET).

## Patentansprüche

1. Absorbierender Artikel mit:
einer rückwärtigen Lage (10), einem absorbierenden Körper (11) und einer oberen Lage (13), die übereinander liegen und einen Verbund bilden;
gegenüberliegenden Endbereichen in Längsrichtung des Verbundes, die jeweils einen ersten und zweiten Taillenbandbereich (16,17) bilden, die derart ausgebildet sind, daß sie einander in der Nähe ihrer seitlichen Enden überlappen, nachdem sie um den Körper des Benutzers herumgeführt wurden;
einer primären, mechanischen Verschlußeinrichtung (27) zum Befestigen der ersten und zweiten Taillenband-Bereiche (16,17) aneinander in der genannten überlappenden Stellung, wobei die primäre, mechanische Verschlußeinrichtung (27) eine erste Komponente (28) aufweist, die in einer ersten Hälfte in Längsrichtung des Verbundes angeordnet und mit dem Verbund an jedem seitlichen, auf die Innenseite des Artikels weisenden Ende des ersten Taillenbandbereiches (16) angeordnet ist, und eine zweite Komponente (21) aufweist, die am Verbund am zweiten Taillenband-Bereich (17) angeordnet ist, wobei die zweite Komponente (21) derart ausgestaltet ist, daß sie mit der ersten Komponente (28) zusammenwirkend in Eingriff tritt und daran lösbar befestigt ist;
**gekennzeichnet durch** eine Kombination mit einer sekundären, mechanischen Verschlusseinrichtung zum Sichern des Verbunds in einem Wegwerf-Zustand nach Benutzung, wobei die sekundäre, mechanische Verschlusseinrichtung eine nach außen gerichtete, dritte Komponente (35) aufweist, die einstückig angeordnet ist und in der ersten Hälfte des Verbunds auf der rückwärtigen Lage (10) aufliegt, wobei die dritte Komponente (35) derart ausgebildet ist, dass sie mit jeder der seitlich angeordneten, ersten Komponenten (28) mechanisch in Eingriff treten kann, um den Verbund im Wegwerf-Zustand zu sichern, wobei jede der ersten Komponenten (28) auf der dritten Komponente (35) aufliegt.

2. Absorbierender Artikel nach Anspruch 1, wobei die erste, mechanische Verschlusseinrichtung enthält:
einen Haken- und Schlaufenverschluss mit zwei ineinandergreifenden Materialien, wobei das eine ein Hakenmaterial und das andere ein Schlaufenmaterial ist;
wobei eine der beiden Materialien die erste Komponente (28) bildet, die an den seitlichen Endbereichen des ersten Taillenband-Bereichs (16) angeordnet ist, und wobei das andere der beiden Materialien die zweite Komponente (28) bildet, die am zweiten Taillenband-Bereich (17) angeordnet ist, um den ersten und den zweiten Taillenband-Bereich (16, 17) aneinander zu befestigen.

3. Absorbierender Artikel nach Anspruch 1 oder 2, wobei die dritte Komponente (35) an der rückwärtigen Lage (10) ortsfest befestigt ist und dort in einer Längsachse (L) zentriert ist.

4. Artikel nach einem vor vorstehenden Ansprüche, wobei die erste Komponente (28) ein polymeres Material vom Hakentyp ist, das einen Biegemodul von 482600 bis 827440 kPa (70000 bis 12000 psi) und eine Shore-Härte im Bereich D-40 bis D-80 aufweist.

5. Artikel nach Anspruch 4, wobei die Shore-Härte etwa D-61 beträgt.

6. Artikel nach einem der vorangegangenen Ansprüche, wobei die erste Komponente (28) ein Hakenmaterial mit einer abweichenden Hakengestaltung aufweist.

7. Artikel nach einem der vorangegangenen Ansprüche, wobei die erste Komponente (28) ein Hakenmaterial mit einer Hakendichte im Bereich von 440 bis 1040 Haken pro Inch² (68 bis 161 Haken pro cm²) aufweist.

8. Artikel nach Anspruch 7, wobei das Hakenmaterial eine Hakendichte von etwa 740 Haken pro Inch² (etwa 114 Haken pro cm²) aufweist.

9. Artikel nach einem der vorangegangenen Ansprüche, wobei die Komponente (28) ein Hakenmaterial mit einer Reihendichte im Bereich von 7,9 bis 23,6 Reihen pro linearem Zentimeter der Breite (20 bis 60 Reihen pro linearem Inch der Breite) aufweist.

10. Artikel nach Anspruch 9, wobei das Hakenmaterial eine Reihendichte von etwa 16 Reihen pro linearem Zentimeter der Breite (etwa 40 Reihen pro linearem Inch der Breite) aufweist.

11. Artikel nach einem der vorstehenden Ansprüche, wobei die zweite Komponente (21) ein Schlaufenmaterial aufweist, das eine Zwei-Stangen-Schlingungs-Maschenware mit Maschenquerreihen im Bereich von 8 - 16 pro Zentimeter (21 bis 41 pro Inch) und Langreihen im Bereich von 10 bis 18 pro Zentimeter (26 bis 46 pro Inch) aufweist.

12. Artikel nach einem der vorstehenden Ansprüche, wobei die zweite Komponente (21) ein Schlaufenmaterial mit einem Kaliber im Bereich von 0,025 bis 0,1 cm (0,01 bis 0,04 Inch) aufweist.

13. Artikel nach einem der vorangegangenen Ansprüche, wobei die zweite Komponente (21) ein Schlaufenmaterial mit einem Grundgewicht im Bereich von 34 bis 102 g/m² (1,0 bis 3,0 oz/jd²) aufweist.

14. Artikel nach einem der vorangegangenen Ansprüche, wobei die erste Komponente (28) ein Hakenmaterial (28) mit einem polymeren Basismaterial mit einer Dicke von 0,0203 bis 0,0254 cm (0,008 bis 0,01 Inch) und einer Hakenausbildung aufweist, die von der Basis zum Scheitel abgeschrägt ist.

15. Artikel nach einem der vorangegangenen Ansprüche, wobei die erste Komponente (28) ein Hakenmaterial (28) mit einem Kaliber im Bereich von 0,089 bis 0,127 cm (0,035 bis 0,050 Inch) aufweist.

16. Artikel nach einem der vorangegangenen Ansprüche, wobei die zweite Komponente (21) ein aus Maschenware gebildetes Schlaufenmaterial mit einer aufrechten und stabilisierten Schlaufengestaltung aufweist.

17. Artikel nach einem der vorstehenden Ansprüche, wobei die zweite Komponente (21) ein textiles Schlaufenmaterial aufweist, das aus synthetischen Fasern gebildet ist, das Textilmaterial enthält Textilfäden, wobei etwa 15 bis 35 % der Textilfäden Fäden mit 1 bis 15 Filamenten pro Faden sind und eine lineare Fadendichte im Bereich von etwa 1,67 bis 3,3 tex (15 bis 30 d) aufweisen, und von denen etwa 65 bis 85 % der Textilfäden Fäden mit etwa 10 bis 30 Filamenten pro Faden und einer linearen Fadendichte im Bereich von etwa 3,3 bis 5,6 tex (30 bis 50 d) sind.

18. Artikel nach einem der vorstehenden Ansprüche, wobei die erste Kompomente (28) und die dritte Komponente (35) zwischen sich eine Mindestscherfestigkeit von etwa 31 g/cm² (200 g/inch²) und eine Mindestabziehfestigkeit von etwa 30 g/cm₂ der Breite (74 g pro linearem Inch der Breite) aufweisen.

19. Artikel gemäß Anspruch 17, wobei die synthetischen Fasern aus Polyäthylenterephthalat (PET) gebildet sind.

## Revendications

1. Article absorbant comprenant :
une feuille de renfort superposée (10), un corps absorbant (11) et une feuille supérieure (13) constituant un élément composite ;
des portions d'extrémité longitudinales opposées audit élément composite fournissant les première et seconde portions de la ceinture (16, 17), respectivement, conçues pour se chevaucher l'une l'autre près de leurs extrémités latérales à l'endroit où elles entourent l'abdomen de l'utilisateur ;
un moyen de fermeture mécanique principal (27) pour attacher lesdites première et seconde portions de la ceinture (16, 17) l'une avec l'autre de manière qu'elles se chevauchent, ledit moyen de fermeture mécanique principal (27) ayant un premier élément (28) situé dans une première demi-portion longitudinale dudit élément composite et attaché audit élément composite en chaque extrémité latérale de ladite première portion de ceinture (16) tournée vers l'intérieur dudit article et ayant un second élément (21) attaché audit élément composite dans ladite seconde portion de ceinture (17), ledit second élément (21) étant construit pour faciliter son imbrication avec ledit premier élément (28) et pour y être attaché de manière lâche ;
caractérisé par une combinaison de ce qui précède avec un moyen de fermeture mécanique secondaire pour attacher l'élément composite lorsqu'il est à jeter après utilisation, ledit moyen de fermeture mécanique secondaire comprenant un troisième élément tourné vers l'extérieur (35) aménagé en un seul élément recouvrant la feuille de renfort (10) de ladite première demi-portion dudit élément composite, ledit troisième élément (35) étant construit pour engager mécaniquement chacun desdits premiers éléments situés latéralement (28) pour attacher l'élément composite lorsqu'il est à jeter avec chacun desdits premiers éléments (28) recouvrant ledit troisième élément (35).

2. L'article absorbant selon la revendication 1 dans lequel ledit moyen de fermeture mécanique principal comprend :
une attache à crochet et à boucle comportant deux matières pouvant s'enchevêtrer, l'une étant une matière à crochets et l'autre étant une matière à boucles ;
l'une de ces deux matières fournissant ledit premier élément (28) attaché aux portions d'extrémité latérales de ladite première portion de ceinture (16) et une autre desdites matières fournissant ledit second élément (21) attaché à ladite seconde portion de ceinture (17), ladite matière pouvant s'engager pour attacher les première et seconde portions de ceinture (16, 17) l'une avec l'autre.

3. L'article absorbant selon la revendication 1 ou 2, dans lequel le troisième élément (35) est attaché de manière fixe à la feuille de renfort (10) et centré sur une axe longitudinal (L) de celui-ci.

4. Un article selon l'une quelconque des revendications précédentes, dans lequel ledit premier élément (28) est une matière polymérique à crochets ayant un coefficient de réduction avec flexion de 482.600 - 827.440 kPa (70.000 - 120.000 psi) et une dureté Shore comprise entre D-40 et D-80.

5. Un article selon la revendication 4, dans lequel ladite dureté Shore est environ de D-61.

6. Un article selon l'une quelconque des revendications précédentes, dans lequel le premier élément (28) comprend une matière à crochets ayant un motif de crochets alternés.

7. Un article selon l'une quelconque des revendications précédentes, dans lequel ledit premier élément (28) comprend une matière à crochets ayant une densité de crochets comprise entre 68 et 161 crochets par cm carré (soit entre 440 et 1040 crochets par pouce carré).

8. Un article selon la revendication 7, dans lequel ladite matière à crochets a une densité de crochets d'environ 114 crochets par cm carré (environ 740 crochets par pouce carré).

9. Un article selon l'une quelconque des revendications précédentes, dans lequel ledit élément (28) comprend une matière à crochets ayant une densité de rangées comprise entre 7,9 et 23,6 rangées par cm linéaire de large (entre 20 et 60 rangées par pouce linéaire de large).

10. Un article selon la revendication 9, dans lequel ladite matière à crochets a une densité de rangées d'environ 16 rangées par cm linéaire de large (environ 40 rangées par pouce linéaire de large).

11. Un article selon l'une quelconque des revendications précédentes, dans lequel ledit second élément (21) comprend une matière à boucles comportant un tissu à mailles tricotées à deux barres ayant des rangées de mailles comprises entre 8 et 16 par cm (soit entre 21 et 41 par pouce) et ayant des colonnes de mailles comprises entre 10 et 18 par cm (soit entre 26 et 46 par pouce).

12. Un article selon l'une quelconque des revendications précédentes, dans lequel ledit second élément (21) comprend une matière à boucles ayant une épaisseur comprise entre 0,025 et 0,1 cm (soit entre 0,01 et 0,04 pouce).

13. Un article selon l'une quelconque des revendications précédentes, dans lequel ledit second élément (21) comprend une matière à boucles ayant un poids de base compris entre 34 et 102 g. par mètre carré (soit entre 1,0 et 3,0 onces par yard carré).

14. Un article selon l'une quelconque des revendications précédentes, dans lequel ledit premier élément (28) comprend une matière à crochets (28) ayant une matière polymérique de base d'une épaisseur comprise entre 0,0203 et 0,0254 (soit entre 0,008 et 0,01 pouce) et une configuration à crochets qui est conique de la base vers l'extrémité.

15. Un article selon l'une quelconque des revendications précédentes, dans lequel le premier élément (28) comprend une matière à crochets (28) ayant une épaisseur comprise entre 0,089 cm et 0,127 cm (soit entre 0,035 et 0,050 pouce).

16. Un article selon l'une quelconque des revendications précédentes, dans lequel ledit second élément (21) comprend une matière à boucles constituée d'un tissu à mailles tricotées ayant une construction à boucles relevée et stabilisée.

17. Un article selon l'une quelconque des revendications précédentes, dans lequel ledit second élément (21) comprend un tissu à boucles constitué de fibres synthétiques, ledit tissu comportant des fils dans lequel environ 15 à 35 % des fils sont des fils ayant 1 à 15 filaments par fil et ayant une densité linéaire de fils comprise entre environ 1 67 et 3,3 tex (15 - 30 d), et dans lequel environ 65 à 85 % des fils sont des fils ayant environ 10 à 30 filaments par fil et une densité linéaire de fils comprise environ entre 3,3 et 5,6 tex (30 - 50 d).

18. Un article selon l'une quelconque des revendications précédentes, dans lequel lesdits premier élément (28) et troisième élément (35) ont une propriété au cisaillement minimum entre eux d'environ 3l g par cm carré (soit 200 g par pouce carré) et une propriété au pelage minimum d'environ 30 g par cm de large (soit 75 g par pouce linéaire de large).

19. Un article selon la revendication 17, dans lequel lesdites fibres synthétiques sont constituées de polytéréphtalate d'éthylène (PETP).
